# EUROPEAN PATENT APPLICATION

(11) **EP 4 268 904 A1**
(43) Date of publication of application: **01.11.2023**
(21) Application number: 22305611.0
(22) Date of filing: 26.04.2022
(51) Int. Cl.: A61Q 19/00, A61K 8/06, A61K 8/55, A61K 8/81, A61K 8/37, A61K 8/23, A61K 8/34, A61K 8/31

(54) **STABLE HIGH INTERNAL PHASE EMULSIONS AND COMPOSITIONS COMPRISING THE SAME**

(71) Applicant: Chanel Parfums Beauté, 92200 Neuilly-sur-Seine (FR)
(72) Inventor: LEE, Seongcheon, SEOUL (KR)
(74) Representative: Plasseraud IP

(57) **Abstract**

High internal phase emulsion comprising at least 74% by volume of an aqueous internal phase, and an oily external phase, wherein the emulsion is stabilized by stabilizing system comprising at least:

(a) lecithin;

(b) a sodium acrylate copolymer ;

(c) a polyglyceryl fatty acid ester, preferably polyglyceryl polyricinoleate,

wherein the stabilizing system represents at least 3.2% by weight of the emulsion.

A process for preparing said high internal phase emulsion by direct emulsification method or by phase inversion method.

Cosmetic compositions comprising said high internal phase emulsion.

## Description

The present invention is directed to a stable high internal phase emulsion (HIPE) and compositions comprising the same.

More particularly, the invention is directed to a stable high internal phase emulsion that is suitable to serve as a base composition for a variety of end use skincare and/or makeup compositions for keratin materials, preferably for the skin. The HIPE of this invention, surprisingly, is particularly stable and can easily be manufactured. The HIPE also displays excellent sensory characteristics such as quick break sensation and silky / lightweight finish.

### BACKGROUND OF THE INVENTION

There are many different products which are designed to be applied to the skin, including creams, liquids and gels. For many cream or gel products, an important factor is the rheological profile of the product. It is common to use products which have a non-Newtonian rheological profile (products which are thixotropic or pseudoplastic). Such products do not run when in packaging and so are easy to handle, but spread easily on the skin's surface when spreading force is applied.

These desirable rheological characteristics can be achieved in a number of different ways. It is important that the rheology remains stable throughout the lifetime of the product. One standard method in the field is to use an oil-in-water or water-in-oil emulsions, which are stabilized using various emulsifiers. However, interesting rheology properties can only be obtained when adding rheological agents such as elastomers to conventional emulsions.

Alternative emulsions providing excellent rheological properties are a high internal phase emulsions (HIPEs). High internal phase emulsions are highly concentrated gelled emulsions with an internal phase volume fraction (Φ) exceeding 0.74. When the internal phase volume fraction exceeds this value, the dispersed droplets reach their maximum packing density and give rise to highly viscoelastic flow behavior. Because of these viscous flow characteristics, HIPEs have gained popularity for numerous applications, including cosmetics

Conventional water-in-oil (W/O) or HIPE emulsions are typically stabilized with silicone raw materials such as silicone oils, silicone elastomers due to their stabilizing functions and lightweight/non-greasy sensory. Consequently, plenty of water-in-silicone (W/Si) emulsions have been globally developed. However, silicones are known to have negative impact on environment, indicating that they are not eco-friendly and lack of sustainability.

It is also known that HIPE emulsions are difficult to manufacture since that have a small amount of external phase (less than 26%). First the difficulty arises when mixing small portion of oil phase in the tank because normally the oil phase stays in the bottom. Secondly, addition of water phase should be done very carefully, dropwise, to prevent phase inversion, which means longer production time and more energy.

In view of the above, there is a significant need to develop HIPE emulsions containing preferably between 80 to 90% of internal phase, that are very stable, easy to manufacture and that display excellent sensory characteristics.

This invention, therefore, is directed to a HIPE, that is stable and that has desirable characteristics when used, for example, as a base for skin care or make-up compositions. The HIPE of this invention is stable for at least one month when stored at room temperature (25°C). Moreover, the HIPE of the invention displays excellent sensory characteristics such as quick break sensation and silky/lightweight finish.

### SUMMARY OF THE INVENTION

The present invention pertains to a high internal phase emulsion comprising at least 74% by volume of a aqueous internal phase, and an oily external phase, wherein the emulsion is stabilized by stabilizing system comprising at least:
(a) lecithin;
(b) a sodium acrylate copolymer ;
(c) a polyglyceryl fatty acid ester, preferably polyglyceryl polyricinoleate,
wherein the stabilizing system represents at least 3.2% by weight of the emulsion.

It is indeed of the merit of the applicant to have discovered that this specific combination of surfactant made it possible to efficiently stabilize HIPE emulsions, without using silicone compounds.

The invention therefore encompasses a high internal phase emulsion comprising less than 0.5% by weight of silicone compounds, preferably less than 0.1% by weight, and more preferably, the emulsion is free of silicone compounds.

The applicant also surprisingly observed that the HIPE (high internal phase emulsion) emulsion of the present invention was easy to manufacture, contrary to conventional HIPE emulsion.

The present invention is therefore also directed to a process for preparing a high internal phase emulsion according to any of the preceding claims by direct emulsification method or by phase inversion method.

The invention also encompasses a cosmetic composition comprising a high internal phase emulsion as described above.

Finally, the invention is also directed to a cosmetic method for skin care of make-up of the skin, comprising the topical application onto said skin of such a cosmetic composition comprising a high internal phase emulsion as described above.

### DETAILED DESCRIPTION OF THE INVENTION

The high internal phase emulsion according to the present invention comprises at least 74% by volume of a aqueous internal phase
(a) lecithin;
(b) a sodium acrylate copolymer ;
(c) a polyglyceryl fatty acid ester, preferably polyglyceryl polyricinoleate,
wherein the stabilizing system represents at least 3.2% by weight of the emulsion.

The HIPE of this invention is stable for at least one month when stored at room temperature (25°C), and preferably at least one month when stored at 45°C.

In a preferred embodiment, the **stabilizing system** represents 3.2 to 10% by weight of the emulsion, preferably 4.2 to 6% by weight of the emulsion.

More particularly, the stabilizing system can preferably comprise:
(a) 0.2 to 4% by weight of lecithin, preferably 0.3 to 1% by weight and more preferably 0.35 to 0.6% by weight;
(b) 1.5 to 5% by weight of sodium acrylate copolymer, preferably 1.6 to 4% by weight and more preferably 1.6 to 2.5% by weight;
(c) 1.5 to 6% by weight of polyglyceryl fatty acid ester, preferably 1.5 to 4% by weight and more preferably 2 to 4% by weight.

In a particularly preferred embodiment, the weight ratio of lecithin: sodium acrylate copolymer: polyglyceryl fatty acid ester will be ranging from 1:4:8 to 1:5:9, and more preferably will be between 1:4:5 - 1:4:7.5.

In a preferred embodiment, the polyglyceryl fatty acid ester is a polyglyceryl polyricinoleate, and in particular, a polyglyceryl-3-polyricinoleate or a polyglyceryl-6-polyricinoleate.

Lecithin will preferably be of natural origin, in particular derived from soybean and/or sunflower seeds.

A mixture of lecithin and sodium acrylate copolymer can for example be available under the commercial reference Lecigel^{®} sold by the company Lucas Meyer Cosmetics.

The HIPE emulsion according to the invention comprises an **aqueous internal phase,** comprising water (preferably demineralized water or floral waters), and optionally any water soluble solvents such as alcohols, in particular ethanol.

The quantity of water in the HIPE emulsion is preferably from 60 to 90% by weight, preferably 65 to 80 by weight, most preferably 68 to 72% by weight of the emulsion.

The aqueous internal phase represents at least 74% by volume of the HIPE emulsion, preferably 80 to 90 vol% of the emulsion.

The HIPE emulsion according to the invention also comprises an **oily external phase,** comprising at least one oil.

The oil should preferably be liquid at room temperature (20°C) and should be cosmetically and pharmaceutically acceptable.

It is possible to employ any vegetable and animal oils, provided that silicon oils are avoided.

Examples of preferred oils are (in decreasing order of preference):
- Fatty esters such as esters of C₈-C₁₀ acids and C₁₂-C₁₈ fatty alcohols (for example coco-caprylate/caparate) or diester of carbonic acid and caprylyl alcohol (for exemple dicaprylyl carbonate)
- fatty acid triglycerides (for example caprylic/capric triglycerides)
- hydrocarbon-based oils of plant origin, such as squalane or linear or branched alkanes comprising from 7 to 20 carbon atoms (for example C15-19 alkane, C9-12 alkane, dodecane and tridecane)
- or mixtures thereof.

The quantity of oily phase in the emulsion is from 5 to 25%, preferably from 5 to 15%, and most preferably from 10 to 14% by weight of the HIPE emulsion.

If the emulsion contains less than 5% of oily phase, it is generally not possible to obtain a stable water-in-oil emulsion, whereas if the emulsion contains more than 25% of oily phase, then the emulsion will cease to exhibit the special properties and characteristics attributable to a high internal phase emulsion.

The present invention is also directed to a **process** for preparing a high internal phase emulsion according to any of the preceding claims by direct emulsification method or by phase inversion method.

Indeed, the present HIPE emulsion has the advantage of being easy to prepare contrary to usual HIPE emulsion, while remaining stable.

Direct emulsification method should be understood as introducing the internal aqueous phase into the external oily phase under stirring. IN this process, the aqueous phase should preferably be added dropwise and slowly to prevent phase inversion.

Inversion method comprises adding the oily phase into the water phase under stirring until a water-in-oil emulsion is obtained. This method is preferred since the oily phase can be added at once: it is then much easier and it saves time.

The HIPE emulsion according to the invention can be employed as a vehicle for a wide variety of cosmetically or pharmaceutically active ingredients, particularly ingredients which have some beneficial effect when applied to the skin.

The emulsion thus provides a means whereby such active ingredients can be diluted, preserved, conveyed to and distributed on the skin surface at an appropriate concentration.

The present invention is therefore directed to **cosmetic compositions** comprising a high internal phase emulsions previously described.

The cosmetic composition according to the invention may be in the form of a skincare and/or makeup composition for keratin materials, preferably for the skin.

In particular, a composition of the invention may be in the form of a skincare composition, in particular in the form of anti-aging, hydrating, whitening cream.

According to another embodiment, a composition of the invention may be in the form of a makeup composition for the face, in particular in the form of a foundation, a makeup base, concealer or a tinted cream.

The cosmetic composition can comprise, at least one usual additive in the field, such as for example at least one compound chosen from an emollient or humectant agent, an oil, an active agent, a colorant, a preservative, an antioxidant agent, an active agent, an organic or inorganic powder, a sunscreen and a perfume.
- One or more **humectant**(s), such as polyols (glycerine, diglycerine, propylene glycol, propanediol, caprylyl glycol, pentylene glycol, hexanediol), sugars, glycosaminoglycans such as hyaluronic acid and its salts and esters, and polyquaterniums such as lipidure PMB.

Said humectant may be present in the composition in an amount ranging from about 0.1 to about 30% by weight, preferably from about 0.005 to about 10% by weight, based on the total weight of the composition.
- One or more **emollient** agent (s) which may be chosen, for example, from esters such as jojoba esters, esters of fatty acids and of fatty alcohol (octyldodecyl myristate, triethylhexanoin, Dicaprylyl carbonate , Isostearyl isostearate, caprylic/capric triglyceride), butters such as shea butters (butyrospernum parkii butter extract, shea butter ethyl esters, marketed under the trade names LIPEX SHEASOFT, LIPEX SHEA-U, LIPEX SHEA, LIPEX SHEALIGHT, LIPEX SHEA TRIS) or moringa (moringa oil/hydrogenated moringa oil esters), waxes (acacia decurrens flower wax & helianthus annuus cera seed seed wax, C10-18 triglycerides), vegetable oils, phytosqualane, alkanes (undecane, tridecane)

Said emollient may be present in the composition in an amount ranging from about 0.1 to about 30% by weight, preferably from about 0.5 to about 10% by weight, based on the total weight of the composition.
- One or more **film-forming** agent(s) such as polymers of natural origin, possibly modified, may be chosen from shellac resin, sandarac gum, gum arabic (ACACIA SENEGAL GUM), dammars, elemis, copals, cellulosic polymers, polymers extracted from the fruit of Caesalpinia spinosa and/or from the algae Kappaphycus alvarezii (such as the product Filmexel^{®}), and mixtures thereof.

Said film-forming agent may be present in the composition in an amount ranging from about 0.1 to about 15% by weight, preferably from about 0.5 to about 10% by weight, based on the total weight of the composition.
- One or more **coloring** agent(s) chosen from pigments, pearlescent pigments, soluble dyes, preferably soluble in water. The pigments may be white or colored, mineral and/or organic. The mineral pigments may be chosen from zirconium or cerium oxides, as well as zinc, iron or chromium oxides, titanium dioxide. The pearlescent pigments may be chosen from titanium mica covered with an iron oxide, titanium mica covered with bismuth oxychloride, titanium mica covered with chromium oxide, titanium mica covered with an organic dye, as well as pearlescent pigments based on bismuth oxychloride. The water-soluble dyes may be chosen from FDC Red 4, DC Red 6, DC Red 22, DC Red 28, DC Red 30, DC Red 33, DC Orange 4, DC Yellow 5, DC Yellow 6, DC Yellow 8, FDC Green 3, DC Green 5, FDC Blue 1.

Said coloring agent may be present in the composition in an amount ranging from about 0.01 to about 20% by weight, preferably from about 1 to about 15% by weight, based on the total weight of the composition.
- One or more **filler**(s) chosen from lauroyllysine, starch, boron nitride and silica

Said filler may be present in the composition in an amount ranging from about 0.1 to about 10% by weight, preferably from about 0.5 to about 7% by weight, based on the total weight of the composition.
- One or more **active** agent(s) of natural, biotechnological or synthetic origin having a biological activity and having an effectiveness on the skin via biological sites, for example chosen from vitamins such as vitamin C and its derivatives (ascorbyl glucoside, 3-o-ethyl ascorbic acid, ascorbyl tetraisopalmitate), vitamin A and its derivatives, vitamin E and its derivatives, vitamin B3 or Niacinamide, panthenol, oligo elements, allantoin, adenosine, peptides (Palmitoyl tetrapeptide-7, Palmitoyl Tripeptide-1, Palmitoyl Pentapeptide-4, Acetyl Dipeptide-1 Cetyl Ester, Acetyl Tetrapeptide-5 marketed under the trade name NP RIGIN, MATRIXYL 3000, IDEALIFT, EYESERYL), plants extracts (glycyrrhiza glabra extract, centella asiatica leaf extract, secale cereal seed extract), yeast extracts, alpha hydroxy acids such as glycolic or lactic acid, tranexamic acid and its derivatives such as cetyl tranexamic ester etc.

Said active agent may be present in the composition in an amount ranging from about 0.01 to about 10% by weight, preferably from about 0.1 to about 5% by weight, based on the total weight of the composition.

In the context of the present invention, the addition of generally unstable active ingredients can be of particular interest. Indeed, it is of the merit of the inventors to have observed that the introduction of generally unstable active ingredient such as vitamin C in the HIPE emulsions of the invention made it possible to surprisingly improve storage stability of these unstable compounds.

Therefore, according to a preferred embodiment, the cosmetic composition comprises active agents, and preferably vitamin C.

Other additives commonly used in cosmetics may also be present in the composition according to the invention, in particular preservatives, antioxidants or perfumes well known in the technical field.

The person skilled in the art is able to choose, among all of these possible additives, both the nature and the quantity of those which will be added to the composition, so that the latter retains all of its properties.

### EXAMPLES

### EXAMPLE 1

The following HIPE emulsions (two according to the invention and two comparative compositions) were prepared though phase inversion method comprises adding the oily phase into the water phase under stirring until a water-in-oil emulsion is obtained.

The stability of the obtained HIPE emulsions where then tested.

| INCI | Ex1 | Ex2 | Ex3 | Comp1 | Comp2 |
|---|---|---|---|---|---|
| | % | % | % | % | % |
| WATER | 69.0 | 68.0 | 68.8 | 71.0 | 71.6 |
| MAGNESIUM SULFATE | 0.5 | 0.5 | 0.5 | 0.5 | 0.5 |
| CHLORPHENESIN | 0.25 | 0.25 | 0.25 | 0.25 | 0.25 |
| GLYCERIN | 10 | 10 | 10 | 10 | 10 |
| PENTYLENE GLYCOL | 3 | 3 | 3 | 3 | 3 |
| **SODIUM ACRYLATES COPOLYMER (82.5%) & LECITHIN (17,5%)** (Lecigel^{®}) | 2.0 | 3.0 | 2.2 | 2.0 | 2.0 |
| **POLYGLYCERYL-6 POLYRICINOLEATE** | 3 | 3 | | 1 | 0.4 |
| **POLYGLYCERYL-** | | | 3 | | |

| **3 POLYRICINOLEATE** | | | | | |
|---|---|---|---|---|---|
| CAPRYLYL GLYCOL | 0.25 | 0.25 | 0.25 | 0.25 | 0.25 |
| DICAPRYLYL CARBONATE | 6 | 6 | | 6 | 6 |
| SQUALANE | 6 | 6 | | 6 | 6 |
| CAPRYLIC/CAPRIC TRIGLYCERIDE | | | 12 | | |
| Emulsion | W/O emulsion | W/O emulsion | W/O emulsion | O/W emulsion | O/W emulsion |
| Water phase (% | 84,75 | 84,75 | 84,75 | 86, 75 | 87,35 |
| Stability in one month at Room Temp | OK | OK | OK | Separation | Separation |

It results from the stability tests demonstrate that the HIPE emulsions of the invention, comprising more than 3% by weight of the stabilizing system of the invention (comprising lecithin, sodium acrylate copolymer and polyglyceryl-6-polyricinoleate) is stable for at least one month at room temperature whereas comparative experiments comprising only 2.4 or 3% of the same stabilizing system were not stable for one month at room temperature.

### EXAMPLE 2

The following HIPE emulsion comprising vitamin C was prepared though phase inversion method and tested for stability

| | |
|---|---|
| INCI | % |
| WATER | 68.8 |
| MAGNESIUM SULFATE | 0.5 |
| CHLORPHENESIN | 0.25 |
| GLYCERIN | 10 |
| PENTYLENE GLYCOL | 3 |
| **SODIUM ACRYLATES COPOLYMER & LECITHIN** (Lecigel^{®}) | 2.2 |
| ASCORBIC ACID | 5 |
| CAPRYLYL GLYCOL | 0.25 |
| **POLYGLYCERYL-3 POLYRICINOLEATE** & | 3 |
| COCO-CAPRYLATE/CAPRATE | 7 |

Acid ascorbic stability was tested through quantitative analysis by UV spectrometer after 1 month storage at 45°C, Room temperature and 5°C.

**Quantitative analysis by UV spectrometer**

| **45°C** | **Room temp** | **5°C** |
|---|---|---|
| **5.1820%** | **5.1645%** | **5.3595%** |

The results surprisingly demonstrate perfect stability of Vitamin C after one month at the 3 temperatures tested. This was unexpected because vitamin C is known to be unstable, in particular at high temperatures.

## Claims

1. High internal phase emulsion comprising at least 74% by volume of an aqueous internal phase, and an oily external phase, wherein the emulsion is stabilized by stabilizing system comprising at least:
(a) lecithin;
(b) a sodium acrylate copolymer ;
(c) a polyglyceryl fatty acid ester, preferably polyglyceryl polyricinoleate,
wherein the stabilizing system represents at least 3.2% by weight of the emulsion.

2. High internal phase emulsion according to claim 1 wherein the aqueous internal phase represents 80 to 90 vol% of the emulsion.

3. High internal phase emulsion according to any of the preceding claims, wherein the stabilizing system represents 3.2 to 10% by weight of the emulsion, preferably 4.2 to 6% by weight of the emulsion.

4. High internal phase emulsion according to any of the preceding claims, wherein the stabilizing system comprises:
(a) 0.2 to 4% by weight of lecithin, preferably 0.3 to 1% by weight and more preferably 0.35 to 0.6% by weight;
(b) 1.5 to 5% by weight of sodium acrylate copolymer, preferably 1.6 to 4% by weight and more preferably 1.6 to 2.5% by weight;
(c) 1.5 to 6% by weight of polyglyceryl fatty acid ester, preferably 1.5 to 4% by weight and more preferably 2 to 4% by weight.

5. High internal phase emulsion according to any of the preceding claims, wherein the oily phase represents 5 to 25 vol% of the emulsion, preferably 8 to 20 vol%, and more preferably 10 to 15 vol%.

6. High internal phase emulsion according to any of the preceding claims, wherein the emulsion comprises less than 0.5% by weight of silicone compounds, preferably less than 0.1% by weight, and more preferably, the emulsion is free of silicone compounds.

7. A process for preparing a high internal phase emulsion according to any of the preceding claims by direct emulsification method or by phase inversion method.

8. Process according to claim 7, wherein the phase inversion method comprises adding the oily phase into the water phase under stirring until a water-in-oil emulsion is obtained.

9. Cosmetic composition comprising a high internal phase emulsion according to any of claims 1 to 6.

10. Cosmetic composition according claim 9, comprising active agents, and preferably vitamin C.

11. Cosmetic composition according to claim 10, in the form of a skin care or a makeup product.
